# EUROPÄISCHE PATENTSCHRIFT

(11) **EP 1 457 160 B1**
(45) Veröffentlichungstag und Bekanntmachung des Hinweises auf die Patenterteilung: **29.04.2009**
(21) Anmeldenummer: 04090081.3
(22) Anmeldetag: 03.03.2004
(51) Int. Cl.: A61B 17/66, A61C 8/00

(54) **Knochen- und Weichteildistraktor**
Bone and soft tissue distractor
Distracteur d'os et des tissus mous

(30) Priorität: 12.03.2003 DE 20304365 U
(43) Veröffentlichungstag der Anmeldung: 15.09.2004
(73) Patentinhaber: Biomed Est., 9490 Vaduz (LI)
(72) Erfinder: Ihde, Stefan, Dr., 8738 Uetliburg (CH)
(74) Vertreter: Radwer, Dieter

(56) Entgegenhaltungen:
- CH-A- 692 898
- DE-A- 10 212 815
- DE-A- 19 948 910
- FR-A- 2 787 698
- US-A1- 2002 156 480

## Beschreibung

Die Erfindung betrifft einen Knochen- und Weichteildistraktor zur Herbeiführung von vertikalem Knochenwachstum, der von der Seite her in einen auf chirurgischem Wege hergestellten Osteotomieschlitz in den Kieferknochen eingesetzt und durch basale Osseointegration im Kieferknochen verankert wird.

In vielen Fällen müssen Implantate an Stellen eingesetzt werden, an denen die vorhandene Kieferknochensubstanz so minimal ist, dass eine ausreichende Verankerung des Implantates im Kieferknochen nicht gewährleistet ist und ein ausreichend dauerhafter und fester Sitz des Implantates nicht erzielt werden kann.

Bekannt sind basalosseointegrierbare Implantate, bei denen die kraftübertragende Basis durch einen oder mehrere voneinander beabstandete Scheibenkörper gebildet wird, wobei die Scheibenkörper einen unterschiedlichen Durchmesser haben können und durch einen in etwa orthogonal zum Fußteil ausgerichteten, durchgehenden Gewindeträger miteinander fest verbunden sind - EP 0 935 949 A1; DE 289 20 487 U1. Die unterschiedlichen Fußteilformen ermöglichen eine weitgehende Anpassung des Implantates an die jeweilige Anatomie des Kieferknochens und sind in der Lage, auch bei einer minimalen, spröden Knochensubstanz höhere Kräfte aufzunehmen und sicher in den Knochen zu übertragen. An sich können diese Implantate auch bei nur noch geringster Knochensubstanz eingesetzt werden und dort erfolgreich funktionieren.

Doch auch wenn es gelingt, den Zahnersatz im Mund zu befestigen, ist manchmal das ästhetische Ergebnis nicht optimal, weil den Weichteilen des Gesichtes nach dem Verlust von Knochen die nötige Knochenunterstützung fehlt. Die Gesichter wirken dann manchmal eingefallen, auch wenn das erste Ziel der Implantation, nämlich die Befestigung von Zähnen, gelungen ist.

Aus DE 199 48 910 A1 ist ein Implantat zum lateralen Einschub in eine gefräste Kieferhöhlung bekannt, das zur Erleichterung der Insertion und Implantierung einen Schaft mit mehreren Gewindezonen aufweist. Diese Gewindezonen tragen scheibenförmige Etagenteile in verstellbarer Anordnung, wobei das unterste Etagenteil, welches die Basisscheibe des Implantates bildet, ebenfalls durch ein Gewinde mit dem Schaft verbunden ist. Diese Entwicklung setzt voraus, dass vor Behandlungsbeginn ausreichender vertikaler Knochen vorliegt, um überhaupt mehrere Etagenscheiben unterbringen zu können.

Distraktoren zur Förderung des Knochenwachstums für die nachfolgende Insertion von enossalen Implantaten sind beispielsweise aus CH 687 672 A5 und DE 299 08 480 U1 bereits bekannt, wobei die vorgeschlagenen Distraktoren nach ihrer Einheilung in den Kieferknochen gleichzeitig die Basis für den späteren Implantataufbau bilden.

Der Distraktor nach CH 687 672 A5 besteht aus einer Folie und einem Träger mit einem säulenartigen Mittelteil, das von einem domförmigen und käfigartig ausgebildeten Gerüst gestützt wird. Durch einen chirurgischen Eingriff werden die den Kieferknochen abdeckenden, weichen Gewebeschichten aufgeschnitten und der Distraktor zur Bildung von Knochengewebe auf den Kieferknochen aufgesetzt. Zu seiner Verankerung auf dem Kieferknochen ist der Distraktor an den freien Enden des Gerüstes mit entsprechenden Spitzen ausgestattet. Nach Ablauf der Distraktionsphase und Bildung einer ausreichenden Knochensubstanz dient das säulenartige Mittelteil, das mit einem Innengewinde versehen ist, bzw. der Distraktor zur Aufnahme und Befestigung der prothetischen Teile.

Nach DE 299 08 480 U1 besteht der Distraktor aus einem oberen zylinderförmigen Kernimplantat, das nach Ablauf der Distraktionsphase als Basisteil für den weiteren Implantataufbau dient, und einem unteren Distraktionteil, das sich bis in die Tiefe des Kieferknochens erstreckt und über ein Distraktionsgestänge, das einen zentralen, mit einem Gewinde versehenen Rundstift aufweist, mit dem oberen Kernimplantat verbunden ist. Durch Drehen des zentralen Rundstiftes werden das obere Kernimplantat und das untere Distraktionsteil gegeneinander verspannt und die Distraktion betrieben, wobei sich das obere Kernimplantat auf das untere Distraktionsteil abstützt.

Alle vorgenannten zylinderförmigen Distraktoren setzen wiederum voraus, dass vor Behandlungsbeginn ausreichend vertikaler Knochen vorhanden ist, damit der Distraktor überhaupt von crestal in den Knochen eingeschoben werden kann und stabil in den Knochen einheilt. Zudem kann die technische Lehre beider Lösungen auf basalosseointegrierbare Implantate, die von der Seite her in ein, auf chirurgischem Wege hergestelltes Implantatbett in den Kieferknochen eingesetzt werden, nicht übertragen bzw. für derartige Implantate nicht angewendet werden.

Eine weitere Distraktionsvorrichtung zur Knochenaugmentation für das nachfolgende Inserieren eines Dentalimplantates ist durch CH 692 898 A5 bekannt. Für die Durchführung der Knochenaugmentation wird auf chirurgischen Wege ein Knochensegment vom Kieferknochen abgetrennt und mittels der Distraktionsvorrichtung sukzessive und inkremental vom Kieferknochen distanziert, um in so entstandenen Trennspalt Knochenwachstum zu initiieren. Die Distraktionsvorrichtung besteht aus einer als Schraubenspindel ausgebildeten Transportschraube, einer Basisplatte, in der die Transportschraube ein- und ausrastbar sowie drehbar gelagert ist und einer Distraktionsplatte, die vom Gewinde der Transportschraube getragen wird. Sowohl die Basisplatte als auch die Distraktionsplatte, die jeweils auf der Trennfläche von Knochensegment und Kieferknochen aufliegen, sind mit Fixierschenkel versehen, die zur exakten Positionierung der Distraktionsvorrichtung und des abgetrennten Knochensegmentes gegenüber dem Kieferknochen dienen und außen am Kieferknochen verankert werden. Der wesentliche Nachteil dieser Lösung besteht darin, das für die Durchführung der Knochenaugmentation ein Knochensegment von einem an sich unverletzten Kieferknochen abgetrennt werden muss. Die Distraktionsvorrichtung dient ausschließlich der Knochenaugmentation und kann als spätere Implantatbasis nicht eingesetzt werden.

Der Erfindung liegt daher die Aufgabe zugrunde, einen Knochen- und Weichteildistraktor zur Förderung des Knochenwachstums für basaloseointegrierbare Dentalimplantate anzugeben, wobei der Distraktor nach Ablauf der Distraktionsphase gleichzeitig als Implantatbasis für die Aufnahme und Befestigung prothetischer Teile verfügbar sein soll.

Erfindungsgemäß wird diese Aufgabe durch einen Knochen- und Weichteildistraktor nach den im Anspruch 1 angegebenen Merkmalen gelöst. Weitere vorteilhafte Weiterbildungen der Erfindung ergeben sich aus den Unteransprüchen 2 bis 15.

Mit der vorliegenden Erfindung wird eine relativ einfache und kostengünstige Lösung für die Distraktion des Kieferknochens zur Förderung des Knochenwachstums in Verbindung mit der Insertion von basalosseointegrierten Implantaten vorgeschlagen.

Die Transportschraube des erfindungsgemäßen Knochen- und Weichteildistraktors mit der die als Distraktionsscheibe dienende crestale Scheibe und die Basisscheibe zur Durchführung der Distraktion voneinander distanziert werden, ist als Gewindeträger ausgebildet, der fußseitige Gewindeabschnitte mit unterschiedlichen Gewindeabmessungen und gegenläufigen Gewindesteigungsrichtungen (Rechts-/Linksgewinde) zur Aufnahme und Distanzierung von Basis- und Distraktionsscheibe aufweist. Der Gewindeträger des Knochen- und Weichteildistraktors, der nach Ablauf der Distraktionsphase gleichzeitig als basalintegriertes Implantat dient, weist kopfseitig vorteilhafterweise bereits einstückig ein Abutment zur Aufnahme und Befestigung der prothetischen Teile (Krone, Brücke, Steg) auf oder ist mit einem entsprechenden Anschlussgewinde zur späteren Verbindung des Abutments versehen.

Die crestale Scheibe und die Basisscheibe, die zusammen mit dem Gewindeträger in den auf chirurgischem Wege hergestellten Osteotomieschlitz von lateral in den Kieferknochen eingesetzt werden, sind mit Seitenflächen versehen, über die die crestale Scheibe und die Basisscheibe bzw. der Knochen- und Weichteildistraktor nach seiner Insertion bikortikal im Kieferknochen abgestützt ist. Gleichzeitig übernehmen die Seitenflächen die Rationssicherung für den nach Abschluß der Distraktion als Implantatbasis dienenden Knochen- und Weichteildistraktor. Für die Insertion des Knochen- und Weichteildistraktors wird lediglich nur ein Schlitz benötigt, über den beide Scheiben in den Kieferknochen eingeführt werden. Hierbei sind die fußseitigen Gewindeabschnitte so ausgebildet, dass die Scheiben bei Insertion einen sehr geringen Abstand zueinander aufweisen oder sich berühren.

Die fußseitigen Gewindeabschnitte können hinsichtlich des Gewindedurchmessers, der Gewindesteigung und der Steigungsrichtung jeweils mit einem unterschiedlichen, voneinander abweichenden Gewinde versehen sein. Dadurch entstehen günstige Voraussetzungen für die durchzuführende Distraktion und für die später nachfolgende EinstellungNeränderung der Bisshöhe. Hierbei sollte aber beachtet werden, dass der Hauptanteil der auftretenden Kaukräfte von der Basisscheibe aufgenommen und übertragen wird. In den überwiegenden Fällen ist es daher zweckmäßig, bei der Auswahl der Gewinde auf den Gewindeabschnitten, das Gewinde, mit dem größere Kräfte übertragen werden können, auf den Gewindeabschnitt anzuordnen, der in die Basisscheibe eingeschraubt wird. Deswegen wird in vorteilhafter Weise die Basischeibe das durchmessermäßig größere Gewinde aufweisen und so das Distraktor-Implantat basal mehr versteifen.

Eine zweckmäßige Ausführungsform besteht darin, dass das Gewinde zur Aufnahme der crestalen Scheibe eine größere Steigung besitzt als das Gewinde, mit dem der Gewindeträger in die Basisscheibe eingeschraubt ist. Hieraus ergibt sich eine größere Hubhöhe der crestalen Scheibe gegenüber der Basisscheibe bei zugleich geringerem Überstand des basisseitigen Gewindes, das basal aus der untere Seite der Basisscheibe hervorsteht.

Nach der Insertion wird durch Drehen des Gewindeträgers die crestale Scheibe, deren Position mittels des Außengewindes auf den Gewindeträger veränderbar ist, gegenüber der Basisscheibe verspannt, wobei zuvor die Kontinuität des crestalen Knochenanteils gegenüber dem basalen Knochenanteil chirurgisch, vorteilhafter Weise im Zusammenhang mit der Einbringung des erfindungsgemässen Distraktors, unterbrochen wurde.

Nach einer kurzen postoperativen Einheilphase von wenigen Tagen kann durch Drehen des Gewindeträgers der Abstand zwischen den Scheiben langsam vergrößert werden, wodurch zwischen den Scheiben und durch die Ausnehmungen in den Scheiben Knochen wächst. Die Distraktion zur Förderung des Knochenwachstums kann so lange weitergeführt werden, bis die Gewindegänge aufgebraucht sind. Je nach der gewünschten Gesamtdistraktionshöhe wird also präoperativ ein Gewindeträger gewählt, der eine ausreichende Gewindelänge aufweist. In der Distraktionsphase stützt sich die als Knochen- und Weichteildistraktor dienende crestale Scheibe über den Gewindeträger auf der Basisscheibe ab. Diese kann in einer besonders vorteilhaften Ausführungsform, bei der die Basisscheibe entsprechend Anspruch 10 über U-förmig ausgebogene Bereiche verfügt, noch rechtwinkelig zur Distraktionsachse abgesichert werden. Diese Absicherungen fixieren die Basischeibe besser im Knochen. In vorteilhafter Weise können jedoch auch Distraktoren mit zwei, fest miteinander verbundenen Basisscheiben verwendet werden.

Nach Ablauf der Distraktionsphase und Erreichen des gewünschten Knochenzuwachses verbleiben die crestale Scheibe und die Basisscheibe oder mindestens die Basisscheibe oder die crestale Scheibe im Kieferknochen und bilden mit dem Gewindeträger die Implantatbasis für die festsitzenden prothetischen Teile.

Die Drehkräfte werden dabei über den Kopf des Gewindeträgers ausgeübt, welcher seitliche Flächen zur Aufnahme eines Drehschlüssels besitzt. Der Kopf kann dennoch getrennt vom Gewindeträger hergestellt sein und erst in der Praxis auf letzterem montiert werden. So können unterschiedliche Köpfe, entsprechend den prothetischen Erfordernissen, gewählt werden.

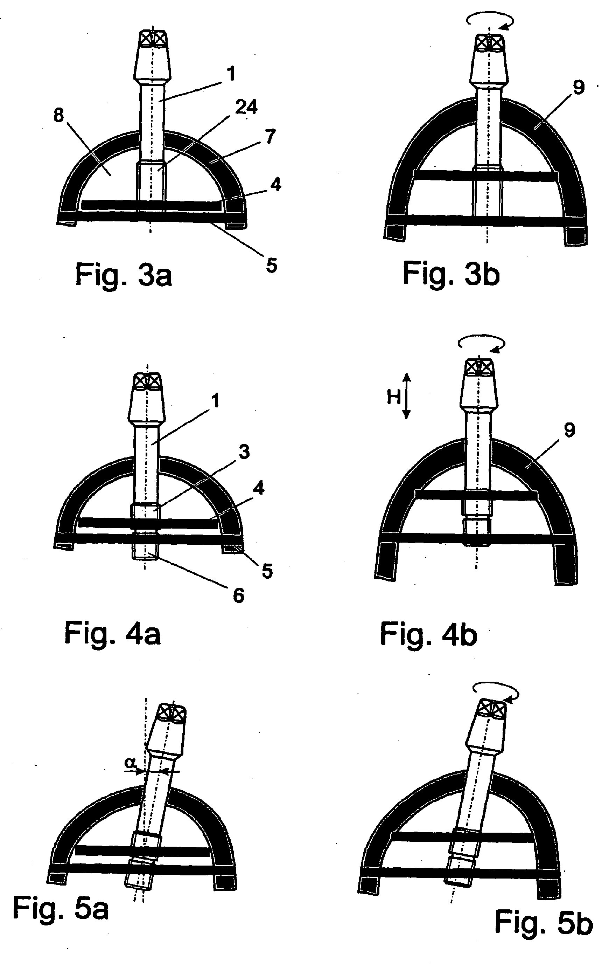

Gemäß einer vorteilhaften Weiterbildung der Erfindung ist der Gewindeträger in der Basisscheibe verdrehbar, jedoch ohne Gewinde angeordnet (Fig. 3a, 3b), während lediglich das Gewinde auf der crestalen Scheibe Gewindeträger dazu dient, den Abstand zwischen der crestalen Distraktionsscheibe und der Basisscheibe allmählich zu vergrößern, um die Distraktion zur Förderung des Knochenzuwachses zu betreiben. Vorteihaft bei dieser Ausführungsform ist, dass die zu Behandlungsbeginn eingestellte Bisshöhe konstant bleibt und durch Verstellen des Gewindeträgers nicht mehr verändert wird.

Mit der Anordnung des Gewindeträgers in der Basisscheibe und der crestalen Scheibe unter einem frei wählbaren Winkel oc (alpha) zur Senkrechten, der zwischen 90° und 45° liegen kann, besteht die Möglichkeit, mit Hilfe des erfindungsgemäßen Knochen- und Weichteildistraktors auch die Richtung des Zuwachses an Knochengewebe zu beeinflussen und zu steuern.

Um die Lagerhaltung bei den Distraktoren zu erleichtern und die Bevorratung zu minimieren, werden die Scheiben und die Gewindeträger separat voneinander in die Praxis geliefert. Der Zahnarzt oder Chirurg kann dann für den jeweils vorliegenden Fall die beste Kombination von Teilen zusammenfügen und implantieren.

Um der unterschiedlichen Knochensubstanz, die bei der Insertion von Implantaten verfügbar ist, Rechnung zu tragen, werden Knochen- und Weichteildistraktoren mit unterschiedlich gestalteten Basisscheiben vorgeschlagen. So kann die Basisscheibe in an sich bekannter Weise durch zwei voneinander beabstandete und miteinander fest verbundene Scheibenkörper gebildet werden, die in der Lage sind, größere Kräfte aufzunehmen und zu übertragen. Generell ist es günstiger, wenn die basale Scheibe größer ist als der crestale Scheibenbereich. Dies deswegen, weil die basale Scheibe dann von crestal her durch Knochen bedeckt ist, was ihre Fixierung und Stabilität im Knochen verbessert.

In der Praxis hat sich gezeigt, dass oberflächenvergrößerte Implantatanteile besser im Knochen fixiert werden können, weil mehr Retention entsteht. Dennoch ist es für den erfindungsgemäßen Distraktor vorteilhaft, wenn der Gewindeträger keinerlei Oberflächenvergrößerung (mit Ausnahme der Gewinde) aufweist, obgleich große Teile im Knochen liegen. Denn durch die Drehung wird der Knochen gereizt, was zu einer unerwünschten Bildung von Bindegewebe führt. Die Scheiben des Distraktors, also die basale und die crestale Scheibe, sind in einer vorteilhaften Ausbildung des Distraktors oberflächenvergrößert. Zum Erreichen dieser Strukturen sind in erster Linie Ätzvorgänge oder Sandstrahlen geeignet oder eine Kombination beider Verfahren.

Es hat sich ferner in der Praxis gezeigt, dass der erfindungsgemäße Distraktor dann zuverlässig und besser als alle bekannten Distraktoren funktioniert, wenn die Scheiben sowohl auf der Einschubseite als auch auf der medialen (runden) Seite kortikal abgestützt sind. Ein wesentliches Merkmal des erfindungsgemäßen Distraktors ist also die bikortikale Abstützung beider Scheiben, die auch die langjährige Funktionsstabilität in der Phase der Funktion als Implantat sichert. Hierzu ist es nötig, dass mindestens auf jeder Seite (einschubseitig bzw. medial) eine Scheibenseite kortikal abgestützt ist. So kann z.B. die basale Scheibe vestibulär abgestützt sein, während die crestale Seite medial (palatinal oder lingual) abgestützt ist. Andererseits reicht es auch, wenn die basale Scheibe medial und einschubseitig kortikal abgestützt ist.

Eine weitere, sehr vorteilhafte Ausführungsform besteht darin, dass die Basisscheibe an der zur Einschubrichtung gegenüberliegenden Seite über eine Biegezone verfügt und der Endabschnitt der Basisscheibe nach der Insertion vestibulär gegen den Kieferknochen abgebogen wird. Der Knochen- und Weichteildistraktor, der nach der Distraktionsphase gleichzeitig als Implantatbasis dient, erhält dadurch eine zusätzliche Verankerung am Kieferknochen und eine zusätzliche Sicherung gegen Rotation.

Eine zusätzliche enossale Abstützung und Sicherung gegen Rotation wird erreicht, wenn die Basisscheibe über einen U-förmig ausgebogenen Endabschnitt verfügt. Diese Lösung hat ferner den Vorteil, dass zur Aufnahme des freien U-Schenkels und zu seiner Verankerung im Kieferknochen ein zweiter Oseotomieschlitz vestibulär in den Kieferknochen eingebracht und nicht, wie bei an sich bekannten Doppel-BOI-Implantaten, ein weiterer Schlitz komplett nach medial durchgefräst werden muss.

Nach einer weiteren, hinsichtlich Lagerhaltung und rationeller Fertigung, vorteilhaften Ausführungsform wird die zusätzliche enossale Abstützung und Sicherung des Knochen- und Weichteildistraktors gegen Rotation durch ein separat hergestelltes Bauteil gebildet, das durch Formschlussverbindung mit der Basisscheibe verbunden ist. Die zur Insertionsrichtung freie Seite der Basisscheibe ist hierzu mit Rastelementen versehen, die von einem Rastkopf des separaten Abstütz- und Sicherungselementes clipartig übergriffen werden, um die Formschlussverbindung herzustellen. Die Lösung hat ferner den Vorteil, dass an die Basisscheibe des Distraktors, beispielsweise in Abhängigkeit von der vorhandenen Knochensubstanz, unterschiedlich ausgebildete Abstütz- und Sicherungselemente angeschlossen werden können.

Bei allen Anordnungs- und Ausbildungsvarianten von crestaler Scheibe und Basisscheibe ist in jedem Fall zu beachten, dass sich die Stege, die die Ringkörper der Scheiben mit dem Gewindeträger verbinden, und die Ringkörper selbst in ihrer peripheren Anordnung nicht überdecken. Hierdurch wird eine bessere Blutzufuhr und Blutzirkulation erreicht, die sich vorteilhaft auf das Knochenwachstum auswirkt und den Einheilungsprozess beschleunigt.

Neben den vorgenannten Maßnahmen zur Verankerung und Sicherung des Distraktors sind die Basisscheibe, die in ihren Breiten- und Längenabmessungen größer ist als die crestale Scheibe und die crestale Scheibe selbst mit Längsflächen versehen, die in Einschubrichtung des Distraktors verlaufen und diesen gegen Rotation, die zu unerwünschten Lockerungen, verbunden mit Entzündungen führen können, sichern.

Der erfindungsgemäße Knochen- und Weichteildistraktor wird vorzugsweise aus Titan, einer Titanlegierung oder aus einer Titan-Molybdän-Legierung, insbesondere aus Ti15Mo, Ti30Mo, Ti6Al4V oder Ti6Al7Nb hergestellt. Diese Legierung besitzen eine höhere Festigkeit, was insbesondere hinsichtlich der Distraktion von Vorteil ist.

Die Erfindung soll nachstehend an einem Ausführungsbeispiel näher erläutert werden. In den dazugehörigen Zeichnungen zeigen:
- Fig. 1: die schematische Darstellung einer ersten Ausführungsform des erfindungsgemäßen Knochen- und Weichteildistraktors im Schnitt,
- Fig. 2: die Draufsicht von Fig. 1,
- Fig. 3a und 3b: eine schematische Darstellung des in den Kieferknochen inserierten Distraktors, mit unveränderlicher Bisshöhe
- Fig. 4a und 4b: die schematische Darstellung des inserierten Distraktors mit der Möglichkeit einer Einstellung der Bisshöhe,
- Fig. 5a und 5b: die Anordnung und Ausbildung des Distraktors zur Beeinflussung der Richtung des Knochenwachstums,
- Fig. 6: eine weitere vorteilhafte Ausführungsform des Knochen- und Weichteildistraktors,
- Fig. 7: die Draufsicht von Fig. 6,
- Fig. 8: die Draufsicht auf die Basisscheibe von Fig. 6
- Fig. 9: die Draufsicht auf die als Distraktor dienende crestale Scheibe
- Fig. 10: eine weitere vorteilhafte Ausführungsform der Basisscheibe des Knochen- und Weichteildistraktors im Längsschnitt
- Fig. 11: die rechte Seitenansicht zu Fig. 10
- Fig. 12: die Draufsicht auf die Basisscheibe nach Fig. 10
- Fig. 13: Die Ansicht in Richtung "A" gemäß Fig. 10

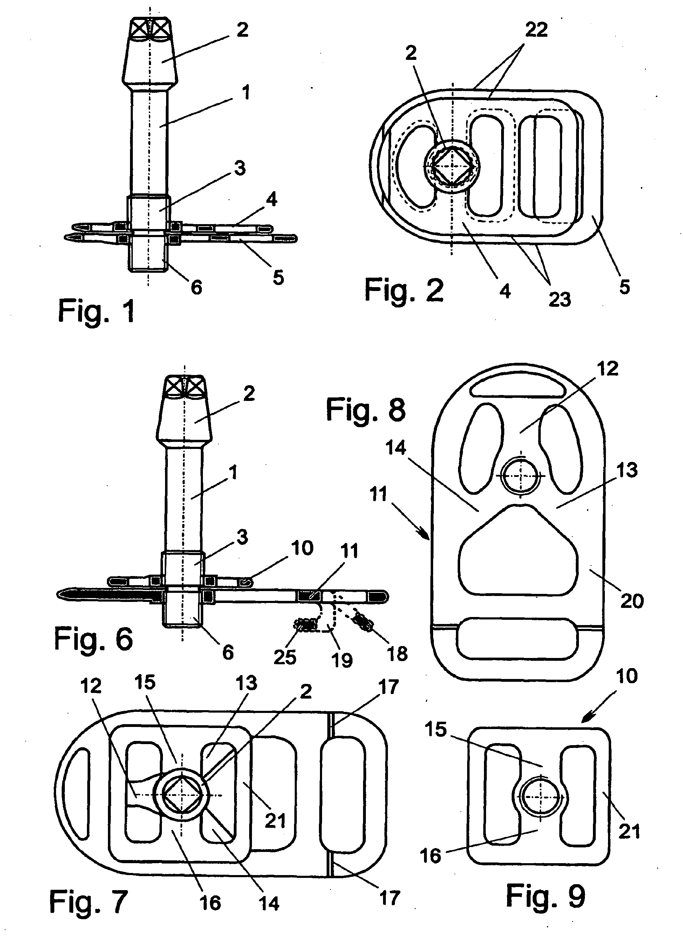

Der erfindungsgemäße Knochen- und Weichteildistraktor besteht aus einem Gewindeträger 1, der fußseitig mit einem Außengewinde ausgestattet ist, das, gemäß dem Ausführungsbeispiel nach Fig. 1 in zwei übereinanderliegende Gewindeabschnitte 3 und 6 unterteilt ist. Der Gewindeträger 1 ist mit dem basalen Gewindeabschnitt 6 in eine Basisscheibe 5 eingeschraubt und trägt auf dem Gewindeabschnitt 3 eine crestale Scheibe 4 zur Durchführung der Distraktion. Der Knochen- und Weichteildistraktor dient nach Abschluss der Distraktionsphase gleichzeitig als Implantatbasis für die Aufnahme und Befestigung der prothetischen Teile, wie beispielsweise einer Krone, einer Brücke oder eines Stegs. Im vorliegenden Beispiel ist der Gewindeträger 1 einstückig ausgebildet und verfügt über ein Abutment 2 zur Aufnahme und Befestigung der prothetischen Teile.

Die Anordnung und Ausbildung der crestalen Scheibe 4 und der Basisscheibe 5, die in ihren Breiten- und Längenabmessungen größer ist als die crestale Scheibe 4, sind so gewählt, dass sich die Ringkörper und die Stege beider Scheiben in ihrer peripheren Anordnung nicht überdecken, um eine gute Durchblutung und Blutversorgung zu gewährleisten. Beide Scheiben verfügen über Seitenflächen 22; 23, die in Einschubsrichtung verlaufen und den Distraktor und die spätere Implantatbasis gegen Rotation sichern und den Knochen- und Weichteildistraktor nach seiner Insertion und bei Durchführung der Distaktion bikortikal im Kieferknochen abstützen.

Der Knochen- und Weichteildistraktor wird in der in Fig. 1 dargestellten geschlossenen Form in einen auf chirurgischem Wege hergestellten Osteotomieschlitz in den Kieferknochen eingesetzt. Nach seiner bestimmungsgemäßen Positionierung wird zur Durchführung der Distraktion mit dem Ziel, das Knochenwachstum zu fördern, durch Drehen des Gewindeträgers 1 der Abstand zwischen der Basisscheibe 5 und der crestale Scheibe 4 allmählich vergrößert. In dieser Distraktionsphase stützt sich die crestale Scheibe 4 über den Gewindeträger auf der Basisscheibe 5 ab, die wiederum enossal verankert ist. - Fig. 3a bis 5b.

Nach der in Fig. 3a und 3b dargestellten Ausführungsform ist der Gewindeträger 1 verdrehbar in der Basisscheibe 5 angeordnet, während die als Distraktor dienende crestale Scheibe 4 zur Durchführung der Distraktion mit Hilfe des Gewindes 24 gegenüber der Basisscheibe 5, wie vorstehend beschrieben, verstellt wird. Die an den crestalen Scheibe 4 und 10 (Fig. 7 und 9) vorgesehenen Seitenflächen dienen hierbei als Führung und Rotationssicherung und verhindern, dass sich die Scheiben 4 und 10 bei Drehung des Gewindeträgers 1 mitdrehen.

Wie anhand von Fig. 4a und 4b deutlich wird, hat die erfindungsgemäße Lösung den Vorteil, dass mit Hilfe des Gewindeträgers 2 und der crestalen Scheibe 4 nicht nur die Distraktion des Kieferknochens durchgeführt werden kann, sondern auch gleichzeitig die Möglichkeit besteht, die Bisshöhe, die symbolisch durch die Höhe H gekennzeichnet ist, nachträglich einzustellen oder anzupassen. Diese Möglichkeit besteht jedoch nicht bei der Lösung nach Fig. 3a und 3b. Bei dieser Ausführungsform bleibt die vorgesehene Bisshöhe bestehen. Es entsteht also unter den Brückenzwischengliedern und vertikal entlang der Gewindeträger neuer Knochen, wobei die Bisshöhe eben durch Verdrehen des Gewindeträgers 1 nicht nachgestellt resp. verändert werden kann. Im Zusammenhang mit der Bildung neuer Knochensubstanz wird jedoch durch die Wirkungsweise der crestalen Scheibe 4 die Kompakta des Kieferknochens angehoben.

Fig. 5a, 5b zeigt in einer Ausführungsform den erfindungsgemäßen Knochen- und Weichteildistraktor, bei dem der Gewindeträger 1 unter einem Winkel ∝ (alpha) zur Senkrechten in die Basisscheibe 5 und in die crestale Scheibe 4 eingeschraubt ist. Durch diese Maßnahme kann die Richtung des Knochenwachstums beeinflusst und gesteuert werden.

Eine weitere vorteilhafte Ausführungsform des erfindungsgemäßen Knochen- und Weichteildistraktors ist in den Fig. 6 bis 9 dargestellt. Als Basisscheibe 5 dient ein Scheibenkörper, dessen Stege 12, 13; 14 in Richtung der beim Einsetzen des Implantates in den Osteotomieschlitz auftretenden Kräfte orientiert sind. Diese Basisscheibe 11 ist dadurch in der Lage, noch größere Kräfte aufzunehmen und zu übertragen. Zur zusätzlichen Verankerung und Sicherung des Distraktors am Kieferknochen 9 verfügt die Basisscheibe 11 über eine Biegezone 17, um den Endabschnitt 18, wie gestrichelt dargestellt, nach Insertion des Distraktors vestibulär gegen den Kieferknochen 9 abzubiegen.

Eine zusätzliche enossale Abstützung und Verankerung des Distraktors und der späteren Implantatbasis im Kieferknochen wird dadurch erreicht, wenn der Endabschnitt 19 der Basisscheibe 11 U-förmig ausformt wird und der freie U-Schenkel 25, gestrichelt dargestellt, in einem zusätzlichen, vestibulär eingebrachten Osteotomieschlitz im Kieferknochen 9 verankert wird.

Auch bei diesen Ausführungsvarianten sind die Stege 12; 13; 14; 15; 16 und :Ringkörper 20; 21 der Basisscheibe 11 und der crestalen Scheibe 10 so ausgebildet und angeordnet, dass sie sich in ihrer peripheren Anordnung einander nicht überdecken, um dadurch eine bessere Blutversorgung zu erreicht und zu sichern.

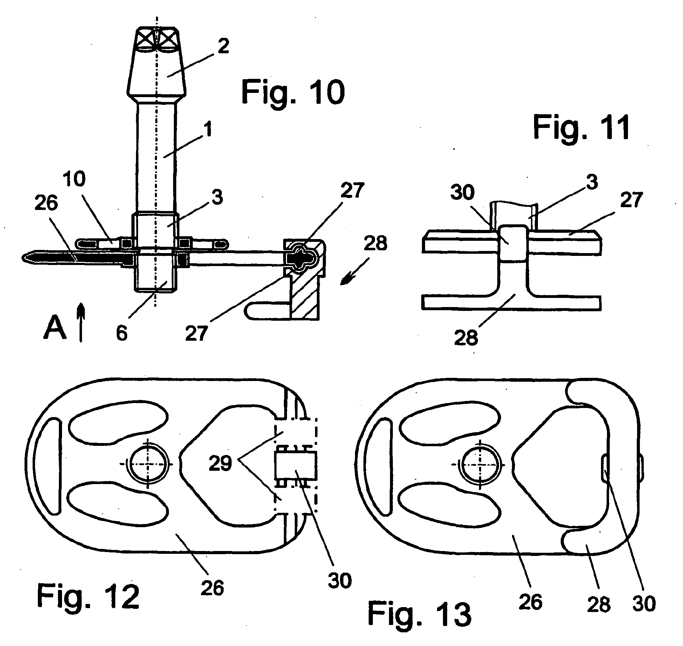

In Fig. 10 bis 13 ist eine weitere, vorteilhafte Ausführungsform des erfindungsgemäßen Knochen- und Weichteildistraktors dargestellt. In dieser Ausführungsvariante ist das zusätzliche Verankerungs- und Abstützelement 28 ein separates Bauteil, das mit des Basisscheibe 26 formschlüssig verbunden ist und wie vorstehend beschrieben, in den vestibulär eingebrachten Osteotomieschlitz im Kieferknochen eingreift. Für die Verbindung von Verankerungs- und Abstützelement 28 und Basisscheibe 26 ist diese mit oberen und unteren Rastelementen 27 versehen, die vom Rastkopf 30 des Elementes 28 clipartig übergriffen werden. Wie insbesondere aus Fig. 12 hervorgeht, kann das Verankerungs- und Abstützelement 28 mit einem Rastkopf 30 oder mit mehreren Rastköpfen 29 ausgestattet sein.

## Patentansprüche

1. Knochen- und Weichteildistraktor mit mindestens zwei Distraktionsscheiben (4, 5) und einem Gewindeträger (1), der von der Seite her in einen auf chirurgischem Wege hergestellten Osteotomieschlitz in den Kieferknochen eingesetzt werden kann und zur Durchführung der Distraktion die Distraktionsscheiben durch Drehen des Gewindeträgers (1), der in einer der Distraktionsscheiben verdrehbar angeordnet ist, voneinander distanziert werden können **dadurch gekennzeichnet, dass** der Gewindeträger (1) fußseitige Gewindeabschnitte (3; 6) mit unterschiedlichen Gewindeabmessungen und gegenläufigen Gewindesteigungsrichtungen, d.h. Rechts-/Linksgewinde, aufweist, während die als Distraktionsscheiben dienende crestale Scheibe (4) und die Basisscheibe (5), Seitenflächen (22; 23) besitzen und nach Insertion über diese Seitenflächen (22; 23) im Kieferknochen (9) bikortikal abgestütz sein können.

2. Knochen- und Weichteildistraktor nach Anspruch 1, **dadurch gekennzeichnet, dass** der Gewindeträger (1) in etwa zentrisch zur Rundung der Basisscheibe (5) oder zur Rundung crestalen Scheibe (4) angeordnet ist.

3. Knochen- und Weichteildistraktor nach Anspruch 1, **dadurch gekennzeichnet, dass** im Gegensatz zum Gewindeabschnitt (6) auf dem Gewindeabschnitt (3) ein Gewinde mit einem größeren Gewindedurchmesser vorgesehen ist.

4. Knochen- und Weichteildistraktor nach Anspruch 1, **dadurch gekennzeichnet, dass** der Gewindeträger (1) am zur Basisscheibe abgewandten Ende einen präformierten Kronenstumpf besitzt, der mit seitlichen Rasten und Flächen versehen ist, in die ein Drehinstrument (Schlüssel) eingreift, wobei der Kronenstumpf und der Gewindeträger zweiteilig ausgebildet sind.

5. Knochen- und Weichteildistraktor nach Anspruch 1, **dadurch gekennzeichnet, dass** der Gewindeträger (2) unter einem Winkel α zur Senkrechten in die Basisscheibe (5) und in die crestale Scheibe (4) eingeschraubt ist und die scheibenseitigen Gewinde schräg eingefräst sind.

6. Knochen- und Weichteildistraktor nach Anspruch 1, **dadurch gekennzeichnet, dass** die Basisscheibe (5) aus zwei voneinander beanstandeten und fest miteinander verbundenen Scheiben- oder Teilscheibenkörpern besteht.

7. Knochen- und Weichteildistraktor nach Anspruch 1, **dadurch gekennzeichnet, dass** die Basisscheibe (5; 11) an der zur Einschubrichtung gegenüberliegenden Seite über eine Biegezone (17) verfügt und der Endabschnitt (19) nach der Positionierung des Distraktors vestibulär gegen den Kieferknochen (9) aufliegend umbiegbar ist.

8. Knochen- und Weichteildistraktor nach Anspruch 1, **dadurch gekennzeichnet, dass** die Basisscheibe (5; 11) eine zusätzliche enossale Abstützung aufweist, die durch einen U-förmig ausgebogenen Endabschnitt (25) gebildet wird.

9. Knochen- und Weichteildistraktor nach Anspruch 1 und 8, **dadurch gekennzeichnet, dass** die zusätzliche enossale Abstützung durch ein separates Verankerungs- und Abstützelement (28) gebildet wird, das mit einer Basisscheibe (26) formschlüssig verbunden ist.

10. Knochen- und Weichteildistraktor nach einem der Ansprüche 1 bis 9, **dadurch gekennzeichnet, dass** die Basisscheibe (5; 11) in ihren Breiten- und Längenabmessungen größer als die crestale Scheibe (4; 10) ist und die Stege der Basisscheibe (5; 11) und der Scheibe (4; 10), die den jeweiligen Ringkörper mit dem Gewindeträger (2) verbinden, so ausgebildet und angeordnet sind, dass sich die Stege und Ringkörper in ihrer peripheren Anordnung nicht überdecken.

11. Knochen- und Weichteildistraktor nach einem der Ansprüche 1 bis 10, **dadurch gekennzeichnet, dass** die Seitenflächen (22; 23) in Einschubrichtung des Distraktors liegen und den Distraktor und die spätere Implantatbasis über die Seitenflächen (22; 23) gegen Rotation gesichert ist.

12. Knochen- und Weichteildistraktor nach einem der Ansprüche 1 bis 11 **dadurch gekennzeichnet, dass** er aus Titan, einer Titanlegierung oder aus einer Titan-Molybdän-Legierung, insbesondere Ti15Mo, oder Ti30Mo, oder Ti6AI4V oder Ti6AI7Nb hergestellt ist.

13. Knochen- und Weichteildistraktor nach einem der vorgenannten Ansprüche, **dadurch gekennzeichnet, dass** eine oder beide Basisscheiben mit einer Oberflächenvergrösserung versehen sind, die durch Ätzen, Sandstrahlen oder eine Kombination der vorgenannten Verfahren hergestellt ist.

14. Knochen- und Weichteildistraktor nach einem der Ansprüche 1 oder 13, **dadurch gekennzeichnet, dass** der Gewindeträger (1) mit Ausnahme der Gewinde (3; 6) keine Oberflächenvergrößerungen oder Strukturen aufweist und völlig glatt ist.

15. Knochen- und Weichteildistraktor nach einem der vorgenannten Ansprüche, **dadurch gekennzeichnet, dass** die Basisscheibe (5) , die crestale Scheibe (4), das Kopfabutment (2) und der Gewindeträger (1) herstellerseitig nicht vormontiert, sondern erst in der zahnärztlichen Praxis nach Bedarf, Knochenangebot und gewünschter Distraktionshöhe zusammenstellbar ausgebildet sind.

## Claims

1. Bone and soft tissue distractor having at least two distraction disks (4, 5) and a threaded part (1), which can be inserted from the side into a surgically prepared osteotomy slot in the jawbone in which the said distraction disks can be separated from each other to carry out the distraction by rotating the threaded part (1) which is arranged rotatably in one of the distraction disks, **characterized in that** the threaded part (1) has at its base threaded sections (3, 6) having different thread dimensions and opposite directions of thread pitch, that is, right-hand and left-hand threads, while the crestal disk (4) and the basal disk (5) that act as the distraction disks have lateral surfaces (22, 23) and can be supported bicortically by these lateral surfaces (22, 23) in the jawbone (9) after insertion.

2. Bone and soft tissue distractor according to Claim 1, **characterized in that** the threaded part (1) is arranged approximately centrally with respect to the curvature of the base disk (5) or to the curvature of the crestal disk (4).

3. Bone and soft tissue distractor according to Claim 1, **characterized in that** a thread having a larger diameter than the thread on thread section (6) is provided on thread section (3).

4. Bone and soft tissue distractor according to Claim 1, **characterized in that** the threaded section (1) on the end away from the basal disk has a preformed crown base with lateral notches or flats into which an instrument for rotating (wrench) is inserted, with the crown base and threaded section made in two parts.

5. Bone and soft tissue distractor according to Claim 1, **characterized in that** the threaded section (2) is screwed into the base disk (5) and into the crestal disk (4) at an angle α from the vertical and the thread at the disk end is cut in obliquely.

6. Bone and soft tissue distractor according to Claim 1, **characterized in that** the base disk (5) comprises two disks or partial disk bodies separated from each other and solidly connected to each other.

7. Bone and soft tissue distractor according to Claim 1, **characterized in that** the basal disk (5, 11) has a bending zone (17) at the side opposite to the direction of insertion and the end section (19) can be bent to lie against the jawbone (9) after vestibular positioning of the distractor.

8. Bone and soft tissue distractor according to Claim 1, **characterized in that** the basal disk (5, 11) has an additional enossal support formed by an end section bent outward in a U shape.

9. Bone and soft tissue distractor according to Claims 1 and 8, **characterized in that** the additional enossal support is formed by a separate anchoring and supporting element (28) mechanically linked to a basal disk (26).

10. Bone and soft tissue distractor according to Claims 1 to 9, **characterized in that** the basal disk (5, 11) has greater lateral and longitudinal dimensions than does the crestal disk (4, 10) and the bars of the basal disk (5, 11) and the disk (4, 10) which connect the particular annular body with the threaded section are made and arranged so that the bars and annular body do not overlap in their peripheral arrangement.

11. Bone and soft tissue distractor according to one of Claims 1 to 10, **characterized in that** the lateral flats (22, 23) lie in the direction of insertion of the distractor and the distractor and the later implant base are secured against rotation by the lateral flats (22, 23).

12. Bone and soft tissue distractor according to one of Claims 1 to 11, **characterized in that** it is made of titanium, a titanium alloy, or a titanium-molybdenum alloy, especially Ti15Mo or Ti30Mo or Ti6AIV or Ti6AI7Nb.

13. Bone and soft tissue distractor according to one of the foregoing claims, **characterized in that** one or both basal disks are provided with a surface enlargement produced by etching, sandblasting, or a combination of the previously named processes.

14. Bone and soft tissue distractor according to one of Claims 1 or 13, **characterized in that** the threaded section (1) has no surface enlargements or structures and is completely smooth except for the thread (3, 6).

15. Bone and soft tissue distractor according to one of the foregoing claims, **characterized in that** the basal disk (5), the crestal disk (4), the end abutment (2) and the threaded section (1) are not preassembled at the factory but rather are designed so that they can be assembled only in the dental practice depending on the requirement, amount of bone, and the desired height of distraction.

## Revendications

1. Écarteur à os et parties molles, avec au moins deux plaques de distraction (4 ; 5) et un support fileté (1) qui peut être introduit par le côté dans une fente d'ostéotomie créée chirurgicalement dans l'os de la mâchoire, les plaques de distraction pouvant être écartées l'un de l'autre pour réaliser la distraction par la rotation du support fileté (1) qui est disposé avec possibilité de rotation dans l'un des plaques de distraction, **caractérisé en ce que** le support fileté (1) présente du côté du pied des parties filetées (3 ; 6) ayant des dimensions de filetage différentes et des directions de pas de filetage opposées, à savoir un filetage à droite ou à gauche, tandis que la plaque crestale (4) et la plaque basale (5) servant de plaques de distraction possèdent des surfaces latérales (22 ; 23) et peuvent s'appuyer sur les deux corticale dans l'os de la mâchoire (9) par ces surfaces latérales (22 ; 23) après l'insertion.

2. Écarteur à os et parties molles selon la revendication 1, **caractérisé en ce que** le support fileté (1) est disposé approximativement au centre par rapport à l'arrondi de la plaque basale (5) ou à l'arrondi de la plaque crestale (4).

3. Écarteur à os et parties molles selon la revendication 1, **caractérisé en ce que**, à la différence de la partie filetée (6), il est prévu sur la partie filetée (3) un filetage ayant un plus grand diamètre de filetage.

4. Écarteur à os et parties molles selon la revendication 1, **caractérisé en ce que** le support fileté (1) possède à l'extrémité opposée à la plaque basale un moignon de couronne préformé, muni de crans latéraux et de surfaces dans lesquelles un instrument de rotation (clé) se met en prise, le moignon de couronne et le support fileté étant construits en deux parties.

5. Écarteur à os et parties molles selon la revendication 1, **caractérisé en ce que** le support fileté (2) est vissé selon un angle a par rapport à la verticale dans la plaque basale (5) et la plaque crestale (4) et les filetages du côté de la plaque sont fraisés en oblique.

6. Écarteur à os et parties molles selon la revendication 1, **caractérisé en ce que** la plaque basale (5) se compose de deux éléments de plaque ou de partie de plaque distants l'un de l'autre et fixés l'un à l'autre.

7. Écarteur à os et parties molles selon la revendication 1, **caractérisé en ce que** la plaque basale (5 ; 11) dispose d'une zone de flexion (17) sur le côté opposé au sens d'insertion et la partie d'extrémité (19) repose de façon à pouvoir être pliée contre l'os de la mâchoire (9) du côté vestibulaire après le positionnement de l'écarteur.

8. Écarteur à os et parties molles selon la revendication 1, **caractérisé en ce que** la plaque basale (5; 11) présente un appui endo-osseux supplémentaire, formé par une partie d'extrémité (25) recourbée en forme de U.

9. Écarteur à os et parties molles selon les revendications 1 et 8, **caractérisé en ce que** l'appui endo-osseux supplémentaire est formé par un élément d'encrage et d'appui (28) séparé qui est relié en correspondance de forme avec une plaque basale (26).

10. Écarteur à os et parties molles selon l'une des revendications 1 à 9, **caractérisé en ce que** la plaque basale (5 ; 11) est plus grande en largeur et en longueur que la plaque crestale (4 ; 10) et les barrettes de la plaque basale (5 ; 11) et de la plaque (4 ; 10) qui relient le corps annulaire correspondant au support fileté (2) sont conformées et disposées de telle manière que les barrettes et les corps annulaires ne se recouvrent pas dans leur disposition périphérique.

11. Écarteur à os et parties molles selon l'une des revendications 1 à 10, **caractérisé en ce que** les surfaces latérales (22 ; 23) se trouvent dans la direction d'insertion de l'écarteur et l'écarteur et la future base d'implant sont fixés pour empêcher leur rotation par les surfaces latérales (22 ; 23).

12. Écarteur à os et parties molles selon l'une des revendications 1 à 11 **caractérisé en ce qu'**il est fabriqué en titane, dans un alliage de titane ou dans un alliage de titane et de molybdène, en particulier Ti15Mo ou Ti30Mo ou Ti6Al4V ou Ti6Al7Nb.

13. Écarteur à os et parties molles selon l'une des revendications précédentes, **caractérisé en ce qu'**une plaque basale ou les deux sont munies d'une augmentation de surface réalisée par attaque chimique, sablage ou une combinaison des procédés susmentionnés.

14. Écarteur à os et parties molles selon l'une des revendications 1 ou 13, **caractérisé en ce que** le support fileté (1) à l'exception des filetages (3 ; 6) ne présente pas d'augmentations de surface ni de structures et est complètement lisse.

15. Écarteur à os et parties molles selon l'une des revendications précédentes, **caractérisé en ce que** la plaque basale (5), la plaque crestale (4), le pilier de tête (2) et le support fileté (1) ne sont pas préassemblés par le fabricant mais sont conçus pour pouvoir n'être assemblés qu'au cabinet dentaire, en fonction des besoins, de l'os disponible et de la hauteur de distraction souhaitée.
